# EUROPEAN PATENT SPECIFICATION

(11) **EP 2 071 329 B1**
(45) Date of publication and mention of the grant of the patent: **29.06.2016**
(21) Application number: 08171049.3
(22) Date of filing: 09.12.2008
(51) Int. Cl.: G01N 33/28, G01N 33/42

(54) **Method for predicting a parameter of a bitumen**
Verfahren zur Prognose eines Bitumenparameters
Procédé pour prédire un paramètre d'un bitume

(30) Priority: 13.12.2007 IT MI20072329
(43) Date of publication of application: 17.06.2009
(73) Proprietor: ENI S.p.A., 00144 Rome (IT)
(72) Inventor: Pavoni, Silvia, 20096 Pioltello (Milano) (IT); Canavesi, Elisabetta, 20124 Milan (IT); D'Elia, Luigi, 26025 Pandino (Cremona) (IT)
(74) Representative: Bottero, Carlo

(56) References cited:
- WO-A1-97/14951
- US-A1- 2002 151 083
- US-B1- 6 477 516

## Description

The present invention relates to a method for predicting a parameter of a bitumen.

The present invention can be inserted within the technical area of prevision methods of the properties of hydrocarbons and, in particular, bitumens.

The term bitumen generally indicates blends of heavy hydrocarbons, having a solid consistency at room temperature, which are normally obtained as vacuum residue from refinery processes, which can be distillation (topping or vacuum) and conversion (thermal cracking and visbreaking) processes of suitable crude oils. They are mainly used for street paving, mixed with appropriate inert materials (crushed stones), but also in the building industry (waterproofing of roofs and foundations). They are commercially classified according to their "normal penetration" (nP) in dmm, by means of a standard needle which penetrates a bitumen sample at 25°C.

Thanks to its resistance to atmospheric agents and its hydrophobic nature, bitumen has long been used for waterproofing applications, such as, for example, roof covering.

The covering materials must have specific chemical and physical characteristics, such as flexibility, break strength at low temperatures, high needle-punching resistance, good rigidity and elongation (to resist the stretching and possible movement of buildings), flow resistance at high temperatures, thermal and dimensional stability, etc.. Of particular importance for a covering material is the minimum susceptibility to temperature effects. It is also desirable for these properties to be maintained following trampling and exposure to atmospheric elements (such as solar light, vapour, atmospheric pollutants).

It is well-known that bitumen itself does not have all these essential properties. For example, bitumen becomes rigid at low temperatures, therefore becoming fragile under said conditions even with respect to light impacts. At high temperatures, on the contrary, the viscosity of bitumen is so low that they spontaneously flows or permanently deforms even due to the effect of minimum mechanical strain. At intermediate temperatures, the viscosity is such to give the bitumen a semi-solid form. This state directly depends on the softening point of a bitumen, i.e. on the temperature at which, conventionally, the bitumen is considered to change from the semi-solid to the liquid state. As a bitumen is made up of numerous and different chemical compounds, it does not have a precise melting or liquefaction point and the change to a liquid state occurs within a range of temperatures. This range is more or less wide, according to the particular physico-chemical structure of a certain bitumen. In order for a bitumen to remain semi-solid at the operating temperature, it is therefore necessary for its softening point to be higher than that temperature. The softening point is determined by means of the Ring-and-Ball method ASTM D36, IP 58, CNR B.U. 35, as described in the embodiment examples of the present invention.

In order to overcome the above drawbacks, it is possible to add modifying components to the bitumen which allow a product having enhanced physical and mechanical properties, to be obtained.

The polymers commonly used for the modification of bitumens include atactic or amorphous polypropylene (PPA), amorphous polyalphaolefins, (APAO), thermoplastic polyolefins (TPO), styrene-butadiene-styrene (SBS), styrene-ethylene-butadiene-styrene (SEBS), synthetic rubber and others.

The incorporation of these modifiers in the bitumen increases the temperature range within which it can be used, as roof covering, improving its mechanical and viscoelastic properties. The use of these modifiers, for example, gives the modified bitumens a higher flow-resistance at high temperature, a higher break strength at low temperatures, a high dimensional stability within a large temperature range, a longer duration and higher resistance to atmospheric agents.

In particular, atactic polypropylene (PPA), a byproduct of the production of isotactic or crystalline propylene (PPI), is a polymer widely used for modifying bitumens for industrial use, both due to its low cost and for its excellent efficacy as a bitumens modifier. The blends of bitumen modified with PPA have, with respect to the blends modified with SBS, better properties at low temperatures, good heat oxidation characteristics and long term resistance to atmospheric agents. The coverings produced by means of these modified bitumens have an excellent resistance to trampling on hot days. Bitumens modified with PPA also have a better UV resistance with respect to the corresponding bitumens modified with SBS.

Bitumens modified with PPA alone, however, do not have a sufficient rigidity and are subject to deformation even as a result of weak mechanical stress, especially at high temperatures. In modifying bitumen, therefore, PPA is normally used together with other components, such as an ethylene-propylene copolymer and PPI. The addition of PPI to a bitumen/PPA blend, gives hardness and rigidity. The addition of an ethylene-propylene copolymer, on the contrary, enhances the flexibility at low temperature. Consequently, depending on the type of bitumen and desired final properties, different modifiers are added to the bitumen, in varying proportions.

Attempts have been made to use modifiers at a lower cost, such as, for example, polyethylene, in order to further reduce the production costs. Roof coverings prepared starting from bitumen modified with polyethylene as primary modifier, however, are not yet available on the market.

The addition of the polymeric modifier (PPA, SBS, etc..) to bitumen is characterized by a so-called phase inversion: the polymer (component in a smaller quantity) englobes the bitumen (component in a larger quantity) forming a continuous phase of a polymeric nature. In correspondence with this phase, the bitumen acquires valuable application properties.

With the other conditions remaining identical, the phase inversion in bitumens modified with PPA, but also with other polymers, takes place as soon as a specific "threshold" content of PPA in the blend is reached, said threshold content being characteristic of the starting bitumen. It can generally be said that the higher the bitumen "compatibility" with PPA, the lower the "threshold" content is. The compatibility of the bitumen with PPA depends on the bitumen composition which, in turn, depends on the mixture of starting hydrocarbons (crude oils and/or distillation residues of crude oils), the transformation processes and the operative conditions adopted.

The bitumens which are most compatible with PPA have the advantage of requiring the use of lower quantities of modifier, thus considerably reducing the preparation costs of the modified bitumen. This leads to the necessity of identifying and selecting the bitumens with a higher compatibility with PPA and from these, consequently, starting crude oils which are more suitable for the production of the bitumen itself. As also mentioned in patent application US2007/0054988 A1, in fact, the characteristics of the modified bitumen vary very significantly depending on the starting crude oil from which the particular type of bitumen to be modified, derives. It is therefore essential to know the chemical composition of the bitumen to determine the levels of saturated hydrocarbons and asphaltenes contained therein.

The phase compatibility of a bitumen with respect to the modifier is therefore an essential property in the field of production processes of modified bitumens, as also mentioned in US2007/0054988 A1, as it represents an indication of the long-term behaviour of the finished product. The phase compatibility, also defined as "networking", also influences other key characteristics of the bitumen/modifier blend such as the viscosity, softening point, penetration at 25°C, low-temperature flexibility, heat stability and long-term heat oxidation. A list of the main properties, and the relative desirable values, for different types of bitumen for industrial applications, at high and low temperatures, is given in Table 1.

**Table 1**

| Bitumen properties | Unit of measurement | Heat applic. | Cold applic. |
|---|---|---|---|
| Phase compatibility | Visual inspection | Very good | Very good |
| Viscosity at 180°C | cP | 7,000 | 4,500 |
| Softening point | °C | 125 | 120 |
| Penetration at 25°C | dmm | 70 | 120 |
| Flexibility at low temperature | °C | -5 | -5 |
| Heat stability | °C | 120 | 120 |
| Low-temperature flexibility after oxidation | °C | +5 | +5 |

The phase compatibility of a bitumen is normally experimentally evaluated by collecting small samples of the modified bitumen, depositing a thin layer of them on a slide and observing the dispersion of the modifying polymer with a fluorescence microscope at 250 magnifications. The image of the sample is normally compared with a graph containing reference images relating to layers of bitumen having different degrees of phase compatibility (the degrees are classified according to an evaluation scale varying from "very good" to "poor"). Each covering manufacturer typically has its own reference graph. In the specific field, however, the phase compatibility evaluations, obtained by means of the above comparison, are relatively uniform.

In the present state of the art, the necessity is therefore particularly felt for effectively and, if possible, rapidly, correlating the nature of the starting crude oil with the properties of the bitumen that can be prepared from this starting crude oil according to a definite production process (for example, its phase compatibility with a certain modifying polymer, the softening point, etc.).

This correlation would allow both the potentialities of the crude oil in the specific field of modified bitumens to be evaluated "a priori" and also to optimize the supply and processing in refineries in which bitumens are produced.

At present, without this correlation, it is possible to predict the potentiality of use of a certain crude oil only by transforming it into bitumen in pilot plants and performing the chemical characterization of the bitumen produced as prototype, all of this of course with a considerable waste of time and with high costs.

The necessity is also currently felt of correlating the nature of the crude oil with the properties of the bitumen (which can be produced starting from said crude oil), such as for example, the phase compatibility with PPA, SBS and other polymers or the softening point, starting from the standard characterization data of the crude oil indicated in the so-called "crude assay".

The crude assay does in fact contain a combination of data deriving from standard physico-chemical characterizations of a specific type of crude oil. These crude assays can be easily found in specialized databases or, alternatively, they can be obtained experimentally by laboratory analyses. Furthermore, in the case of crude hydrocarbon blends, it is also possible to obtain these characterization data by calculation models (so called blending models), which allow the characteristics of a blend of hydrocarbons to be predicted starting from the characterization data of the single components.

The necessity of predicting the properties of bitumens starting from the information available for the crude oil and/or for the starting residues used for producing said bitumens, is even more evident, considering
- the increased variability of the crude oils processed in refineries and the consequent impact this variability has on the quality of finished bitumen;
- the necessity of optimizing the blend of crude oils to be supplied, considering both the quality of the bitumen produced and also the quality of the other products obtained, also keeping in mind the range of yields of the different phases of the overall production process;
- the necessity of avoiding an iterative process on a pilot plant in order to obtain correlation data relating to the bitumen;
- the possibility of exploiting the standard characterization data of the crude oils which are easily available, or which can be produced in the laboratory, and normally also used for optimizing the supply and overall production of refineries.

In the present state of the art, various prediction models of properties of complex blends of hydrocarbons, as also of bitumens, are known and used, based on NMR characterization data, or Near Infrared spectroscopy or other advanced techniques. These data are not normally available in a crude assay of a crude oil and it is therefore necessary for complex analyses to be performed each time, in addition to the availability of a physical sample of the hydrocarbon to be used as raw material.

The document WO97/14953 describes, for example, a method for predicting the physical properties of a hydrocarbon material, such as the residues that can be obtained from crude oils, fuel oils and bituminous compounds, which comprises:
a) selecting a set of physical samples of hydrocarbon residues having different qualities;
b) determining the physical property to be predicted, on various samples, by means of conventional measurements;
c) registering the near infrared spectrum of the various samples;
d) selecting a range of wave-lengths and the use of the relative absorbances as input data for multivariate statistical analysis or for a neural network;
e) correlating the absorbances determined in d) with the physical property determined in b) by means of multivariate statistical analysis or neural networks for generating the model;
f) applying the predictive model starting from the near infrared spectrum of an unknown sample to estimate the physical property according to b).

This method however has the following disadvantages:
- it requires a near infrared spectroscopic analysis directly on the sample of bitumen whose physical property is to be predicted. This analysis is not included in standard analyses for crude oils or for bitumens and is not available in crude assays;
- although it can be potentially used for creating a correlation between near infrared spectroscopic properties of the sample of bitumen and physico-chemical properties of the same bitumen, such as compatibility with PPA, for example, it does not generate a correlation between the properties of the starting crude oil and those of the bitumen produced therefrom according to a certain process method. A similar prediction method based on near infrared spectroscopic analysis is used in document WO97/14951 in a process for preparing a bitumen composition by blending different streams of bitumen.

The document WO 02/47460 A2 describes a method for selecting a crude oil as raw material for the production of paving grade bitumen, by analyzing physical samples with a high resolution mass spectrometer. Also in this case, a non-conventional analytical method is used and the physical presence is necessary of the sample obtained starting from crude oil, by means of distillation under particular temperature and pressure conditions. The method, which is specific for the production of paving grade bitumen by distillation, requires the construction of a specific database with the experimental data obtained by means of mass spectroscopy analysis and does not take into consideration either the indefiniteness of the production processes, such as for example thermal processes which alter the chemical structure and require onerous pilot operations for producing a physical product sample, or other uses or characteristics of the bitumen produced, for example compatibility with PPA.

The document US 6,477,516 B1 defines a method which, starting from NMR analysis of a physical hydrocarbon sample, predicts the desired property for the same product by means of a neural network which has been subjected to appropriate training. The document shows how modelling with neural networks, based on NMR analysis of the product, is effective in also predicting other properties, but does not solve the technical problem faced by the present invention, not responding to the objectives.

From an analysis of the state of the art, a method has not been found which is capable of correlating the characteristics of a crude oil with the properties of a bitumen deriving therefrom, such as, for example, its compatibility with polymeric modifying agents or the softening point. Even less, a method has been found that allows the properties of bitumens to be predicted, starting from standard characterization data of crude oils and/or distillation residues of crude oils easily available in specialized databases or which can be alternatively obtained experimentally via laboratory analyses.

A first objective of the present invention is therefore to define a method capable of correlating the physico-chemical parameters characteristic of a hydrocarbon and the properties of the finished bitumen obtained starting therefrom, such as the phase compatibility between said bitumen and polymeric modifying agents, in particular PPA and SBS, or the softening point.

A second objective of the present invention is to define a method capable of obtaining the above correlation starting from data available in crude assays of crude oils and/or other types of hydrocarbons which can be used for producing bitumens (for example distillation residues of crude oils).

An object of the present invention therefore relates to a method for predicting a parameter of a bitumen having a penetration nP at 25°C, said bitumen being obtained by means of a process (P) applied to a hydrocarbon and/or a blend of hydrocarbons, according to claim 1.

In describing the method according to the present invention, reference will be made to the following figures:

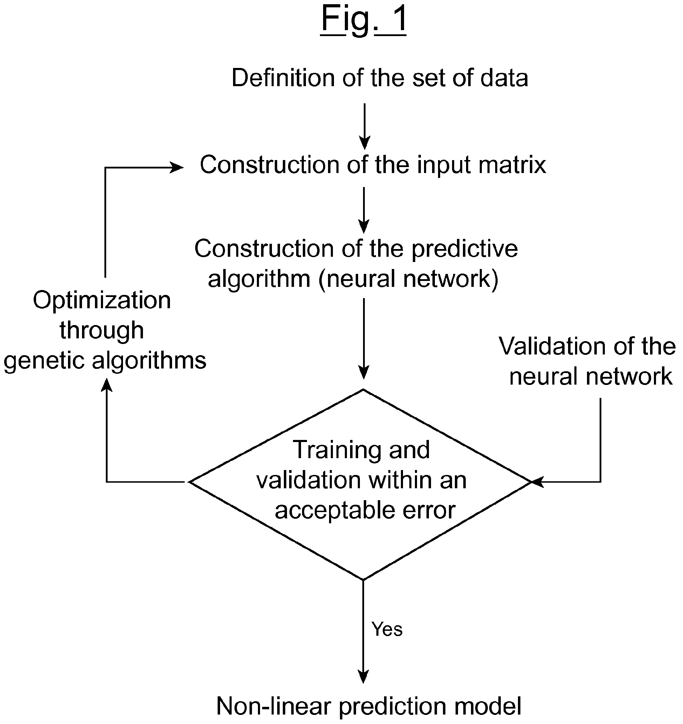

- figure 1: schematic illustration of the arrangement and training procedure of a neural network for the purposes of the present invention;

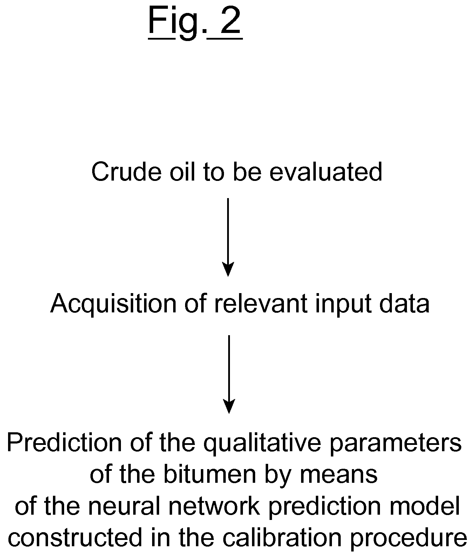

- figure 2 schematic illustration of the functioning in predictive mode of a neural network for the purposes of the present invention.

The method according to the present invention allows a parameter to be predicted, which is characteristic of a bitumen, such as for example its phase compatibility with polymer modifying agents (for example PPA, SBS, etc.) or the softening point, starting from physico-chemical data of the starting hydrocarbon from which the bitumen is obtained, and the penetration value at 25°C (nP) of the bitumen obtained with the process (P) from said starting hydrocarbon.

The method according to the present invention is preferably applied for predicting a phase compatibility index of a bitumen with PPA, such as the phase inversion point.

The method of the present invention, however, as an expert in the field can easily understand, can also be applied to the prediction of other physico-chemical parameters characteristic of a bitumen, in addition to those already cited.

The phase compatibility of the bitumen with PPA is evaluated on the basis of the value of an index, preferably on the basis of the value of the phase inversion point of the bitumen/PPA blend.

The method according to the present invention can also be used, however, for correlating the compatibility of the bitumen with polymers different from PPA, to the physico-chemical characteristics of the starting hydrocarbons from which the bitumen can be obtained through a certain production process.

The phase inversion point of the bitumen/PPA blend, corresponding to the weight percentage of PPA with respect to the total weight of the bitumen/PPA blend, necessary for obtaining phase inversion, is generally determined experimentally by means of the following method. The same method can be used for determining the phase compatibility of the bitumen with other modifying agents of the polymeric type such as SBS. 13% by weight of PPA are added, under standard temperature conditions (185°C), to a known quantity of bitumen, the whole mixture being maintained under stirring for 1 hour. At the end of this period, a drop of bitumen/PPA blend is removed and observed under a fluorescent microscope to observe whether possible phase inversion has taken place. In the affirmative case, the test must be repeated starting from a new blend with a known quantity of bitumen, adding 10% of PPA; if negative, a further 1% of PPA is added to the bitumen/13% PPA blend and the control is repeated after 30 minutes. The test continues with successive additions of PPA until the concentration at which inversion takes place, is reached. If the inversion does not take place even after a maximum addition of 24% of PPA, the test is suspended. Starting from values higher than 17% of PPA, in fact, it becomes increasingly more difficult to produce a modified bitumen under economically acceptable conditions and for values of around 24% of PPA the determination of the inversion point requires relatively long times (more than 1 day).

The higher the phase compatibility of the bitumen with PPA, the lower the inversion point will be, i.e. the quantity of PPA to be added to allow the phase inversion to take place.

In order to determine the phase inversion point, the "phase inversion" in a bitumen/ PPA blend is considered to take place when the image of a sample of the blend, obtained at a fluorescent microscope, has the following two characteristics:
- the presence of a continuous polymeric phase (white field) and a discontinuous bituminous phase in spheroidal form (black drops),
- homogeneous dispersion of the drops of bitumen in the polymeric phase (absence of filaments, elongated spots, etc.).

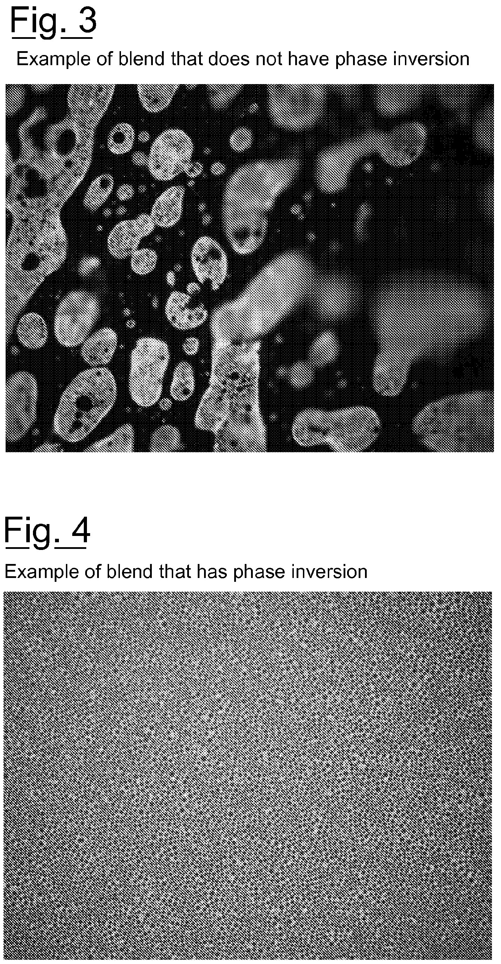

Figures 3 and 4 show, for illustrative purposes, two images at the fluorescence microscope which respectively illustrate a blend which does not have phase inversion and a blend with phase inversion.

For the purposes of the description of the present invention, the starting hydrocarbon sent to the production process (P) of bitumen is a crude oil or a residue deriving from the processing of crude oil (for example distillation residues) and/or mixtures thereof. The term process (P) indicates the processing process of the hydrocarbon for producing a bitumen and the relative operating conditions which determine the characteristics of the finished product i.e. the bitumen. With variations in these conditions, the bitumen obtained starting from the same hydrocarbon can have different physico-chemical characteristics.

The penetration at 25°C is a key parameter linked to the composition of the bitumen and consequently also to the physico-chemical characteristics depending on the composition, such as for example, the phase compatibility of the bitumen with modifying polymers such as PPA or SBS or the softening point. As also specified in US 2007/0054988 A1, the penetration at 25°C nP, determined through the so-called needle penetration method is an index of the softness or hardness of the material and therefore also of its applicative characteristics. The penetration at 25°C is also an important factor to be considered in the installation of roof coverings. A bituminous blend which is too soft (high nP value), for example, can cause problems associated with the practicability of the covering and the formation of marks on its surface in hot climates. A bituminous blend which is too rigid (low nP value), on the other hand, can break in cold climates.

The penetration value at 25°C can be varied, according to techniques known to experts in the field, to adapt it to the specific destinations and climatic conditions of use.

Considering the relation existing in a bitumen between the penetration at 25°C and its phase compatibility with a polymeric modifying agent, and keeping in mind that different penetrations can be required depending on the seasonal or processing conditions, the penetration value at 25°C is the starting parameter characteristic of bitumen to be used in the method according to the present invention.

In addition to the penetration at 25°C, values of other N physico-chemical parameters of the hydrocarbon or blend of starting hydrocarbons, which can be obtained through experimental analyses, databases and/or blending models, must be available for being able to effect the method according to the present invention.

To be able to adequately represent the bitumen whose desired parameter is to be predicted, in physico-chemical terms, N is an integer preferably varying from 3 to 10, more preferably from 4 to 7.

These values, together with the penetration value at 25°C of the bitumen (input data) are fed to a neural network prediction system, which has been subjected to a training phase and preferably optimized for calculation by means of genetic algorithms.

The neural network is trained to correlate the same N physico-chemical parameters of a hydrocarbon, or blend of hydrocarbons, and the nP values of the bitumen to the parameter to be predicted.

On the basis of the correlation between the parameters established during the training phase, the neural network returns the value of the desired parameter (output data). If, for example, the phase compatibility of a bitumen with a polymeric modifying agent (for example PPA or SBS) is to be predicted, the parameter returned can be the value of the relative phase inversion point.

The neural network can consist of a variable number of layers of neurons depending on the type of bitumen considered. It has been observed that generally a network with a maximum number of three layers of neurons can give good correlation results for any type of bitumen.

The transfer function for each parameter to be modelled with the neural algorithm can be linear or nonlinear, depending on the relations between input and output for each layer, and also regardless of the number of layers.

For the training of the neural network for the purposes of the present invention, any training system or software among those known to experts in the field, also commercially available, can be used.

The training phase of the neural network is effected by feeding, as input data, a matrix of values to the neural network.

In order to build the matrix for the training of the neural network, a set of samples consisting of number I of hydrocarbons and/or a number MI of hydrocarbon blends, is defined. The term set of samples refers to a set of samples of different hydrocarbons (crude and/or residue) or a set of various samples of the same hydrocarbon having different nP values in the relative non-modified bitumen produced starting from this. In the case of a set of samples of the same hydrocarbon, in order to obtain an adequate predictive model, it is preferable to have a minimum number of 20 samples.

The numbers I and MI are integers, which can be the same or different, preferably varying from 10 to 90.

For each of the I and/or MI samples a number N' of physico-chemical parameters are determined using standard analytical methods.

N' is an integer preferably varying from 3 to 10, more preferably from 4 to 7.

Among the possible physico-chemical parameters of the starting hydrocarbon, those preferred are density, sulphur content and/or metals content and/or asphaltenes content, viscosity, CCR (carbon residue, determined according to the Conradson method, or according to the Microcarbon test method or according to the Ramsbottom method); with respect to the bitumen, the physico-chemical parameter is penetration at 25°C (nP).

The information on the statistically relevant data, obtained by means of a correlation analysis, is then combined with the corresponding values of the parameter to be predicted (for example phase compatibility index, softening point, etc.) of each of the bitumens resulting from the application of the process (P) to the I hydrocarbons and/or MI blends of hydrocarbons, to generate a matrix of parameters to be used as a basis for the neural network training. The values of the desired parameter are determined experimentally through standard analysis.

During the training process of the network, the initial random weights associated with the input parameters are recalculated and optimized after each iteration process until the error is significantly lower than a preestablished acceptability value for the specific parameter to be predicted.

At the end of the training, the neural network is validated, by testing the accuracy of the output results with an adequate number of unknown samples to verify that the response is within an acceptable prevision standard.

During the construction of the neural network, the optimization phase, i.e. convergence towards the best network, is preferably obtained by means of genetic algorithms, defined as such as part of their terminology derives from genetics.

The genetic algorithms, starting from a selected number of possible solutions (individuals) called populations, allow them to evolve during the realization of the optimization process. With each iteration, the algorithms effect a selection of individuals of the current population, using them for generating new elements of the same population, which will substitute an equal number of individuals already present, thus forming a new starting population for the following iteration (or generation). This succession of generations evolves towards an optimum solution of the assigned problem. The above evolution is obtained through a partial recombination or crossover of the solutions, in which each individual transmits part of its own genetic patrimony to its descendants, and through the introduction of random mutations in the starting population, thus sporadically forming individuals with characteristics not included in those present in the genetic dowry of the original species.

At the end of the evolution phase, the population of the solutions is analyzed and only the solutions which better solve the problem are kept: the individuals with the most suitable qualities for the environment in which they are situated, therefore, have a greater possibility of surviving and reproducing themselves. These solutions will undergo a new evolution phase and so forth.

In the final phase of the evolution process, an optimized population of solutions is obtained for the best solution of the proposed problem, compatibly with the initial input data. The genetic algorithms are normally used for optimization problems for which algorithms having a linear or polynomial complexity are not known.

More specifically, the optimization process preferably consists of the following successive phases:
1. encoding of a structure, in the form of a string, called chromosome or genome (genetic representations of neural networks), i.e. of a set of parameters which define an individual or solution proposed for the problem that the genetic algorithm is attempting to solve;
2. definition of a function capable of restoring the "good quality" of a genome as a solution of the problem. This function is indicated as a fitness function, and is a particular type of objective function;
3. generation, randomly, of an initial population of individuals;
4. selection of a portion of the existing population to generate a new generation of individuals. The individual solutions are selected through a process based on the fitness function, in which the solutions with the highest score (measured through the fitness function) are more likely to be selected;
5. generation of a further population generation of solutions from those selected through genetic operators by crossover or recombination (suitably combining the characteristics of a pair of parents) and/or mutation (effecting random changes on a single parent). This process is aimed at modifying the population, in an attempt to improve it so as to obtain an even better solution;
6. classification of the individuals on the basis of the result of the fitness function;
7. substitution of the parents with the individuals thus generated, allowing the formation of the subsequent generation.

At the end of step 7, the process returns to step 4 and is re-iterated until a process-end criterion is satisfied. Through this process, the solutions, i.e. in this case the best neural networks, survive, their characteristics are transmitted to the future generations combined with other characteristics to find even-improving networks for the specific application.

This genetic research is more effective than a random research, as the genetic recombination process of the characteristics improves the rate for identifying neural networks with good predictive capacities. It also has the potential advantage of being complementary and synergic to the experience of the user, of which the initial assumptions deriving from a knowledge of the parameters are necessary.

Once an adequate training level has been reached, the optimized and validated neural network is ready for use (predictive mode).

The method according to the present invention therefore comprises, between phase a) and phase b), the following additional phase:
a1) preparing a neural network and training it in order to obtain a trained neural network.

The training of the neural network comprises the following operative phases:
i) obtaining by means of experimental analyses and/or databases and/or blending models, the values of N' physico-chemical parameters of a number I of hydrocarbons and/or a number MI of hydrocarbon blends;
ii) obtaining by means of experimental analyses the penetration values at 25°C nP of each bitumen resulting from the application of the process P to the I hydrocarbons and/or MI blends of hydrocarbons;
iii) obtaining by means of experimental analyses the value of the parameter which is to be predicted for each bitumen resulting from the application of the process P to I hydrocarbons and/or MI blends of hydrocarbons;
iv) building an input matrix relating to the I hydrocarbons and/or MI blends of hydrocarbons comprising the values obtained in phases i) to iii),
v) training the neural network with the input matrix obtained in phase iv) to obtain a correlation between the physico-chemical parameters of the I hydrocarbons and/or MI blends of hydrocarbons and the nP values of the bitumens resulting from the application of the process P to the I hydrocarbons and/or MI blends of hydrocarbons, and the parameter values to be predicted, thus obtaining a trained neural network.

For arranging the network and its training, the parameters to be predicted which can be preferably used are the phase compatibility of the bitumen with a polymeric modifying agent (for example PPA, SBS, etc.) or its softening point.

The method according to the present invention has various advantages with respect to the state of the art. First of all, it allows the properties of bitumens to be predicted starting from easily available data relating to the physico-chemical characteristics of the hydrocarbons (residues and/or crude oils) to be processed and from the penetration value of the finished bitumen. The method allows reliable prediction data to be rapidly obtained, avoiding having to resort to lengthy conventional laboratory analyses.

The method allows prediction data to be obtained for parameters of fundamental importance from the point of view of practical applicability of bitumens, such as phase compatibility with various kinds of polymeric modifying agents or the softening point.

Secondly, the method according to the present invention allows the rapid and economic evaluation of the potentiality of crude oils for which there is a standard characterization to be used profitably in the production of bitumens. The standard characterization data are easily available, as they are normally used by refinery operators for managing the supply of raw materials and optimizing refinery processes.

The present invention is also a predictive instrument of the characteristics of bitumens in the case of the use of blends of crude-oils as starting hydrocarbons, as most frequently occurs in refineries, rather than a pure crude-oil. Calculation models (blending models) are in fact known and commonly used, which allow the characteristics of blends of crude-oils to be evaluated, thus also providing physico-chemical characterization data for complex blends from which to start for predicting the phase compatibility of the bitumen.

Finally, the method according to the present invention can also be applied for predicting the properties of bitumens produced starting from new crude-oils, for which, even if there are not yet any standard characterization data, it is possible to effect specific analytical characterizations in the laboratory, as in the case of distillation residues of crude-oils and/or blends thereof. Furthermore, in the case of blends, it is always possible to use blending models for predicting their characteristics starting from the characterization data of the single components.

The following embodiment example is provided for purely illustrative purposes of the present invention and should in no way be considered as limiting the protection scope defined by the enclosed claims.

### Example 1

A neural network consisting of three layers of neurons was trained through a training matrix containing data relating to 69 bitumens. The bitumens were obtained with a process (P) which envisages thermal cracking transformations.

For each bitumen, the following 4 physico-chemical parameters relating to the residue 420+°C of the blend of starting crude-oils, were determined and inserted in the matrix, as input data: API density, viscosity @50°C as Viscosity Blending Number (VBN), percentage content of sulfur and asphaltene content, in addition to the penetration at 25°C of the final bitumen. The values of the percentage content of PPA, at phase inversion, for each bitumen were also inserted in the matrix as output data.

Table 2 indicates the experimental range considered (i.e. the minimum value and maximum value for each parameter in the 69 samples and the relative average) and the results obtained with the neural network in the training phase of the neural network itself (see last column table 2).

The trained neural network was subjected to a subsequent validation of the correlation algorithm. For this purpose, the network was used in predictive mode by feeding, as input data, the data relating to 19 unknown samples of bitumen.

The same physico-chemical parameters used for the training were adopted as input data, excluding the values of the percentage content of PPA at phase inversion for each bitumen. The results relating to the percentage of PPA at phase inversion, returned by the neural network, were compared with the corresponding values determined experimentally. As can be observed in Table 3, on an average the absolute error, expressed as difference between the value measured experimentally and that calculated by the neural network, in the estimation of the inversion point, is lower than 0.7, an extremely good value considering that the repeatability of the analytical method used is 1.

On the basis of this prediction for the specific process, it is possible to effect the ranking of the crude-oils and blends.

### Example 2

A neural network consisting of three layers of neurons was trained through a training matrix containing data relating to 36 bitumens. The bitumens were obtained with a process (P) which envisages thermal cracking transformations.

For each bitumen, the following 4 physico-chemical parameters relating to the residue 420+°C of the blend of starting crude-oils, were determined and inserted in the matrix, as input data: API density, viscosity @50°C as Viscosity Blending Number (VBN), percentage content of CCR and asphaltene content, in addition to the penetration at 25°C of the final bitumen. The values of the softening point for each bitumen were also inserted in the matrix as output data.

The softening point, which conventionally represents the temperature at which a bitumen passes from a semi-solid state to the liquid state, is measured with the Ring-and-Ball method, ASTM D36, IP 58, CNR B.U. 35 briefly described hereunder.

A test-sample of bitumen being tested, placed inside a special brass ring and loaded at the centre with a steel ball having certain dimensions and weight, is introduced into a bath which is heated with a gradient of 5°C per minute. With the increase in temperature, the bitumen, under the weight of the ball, is deformed, becoming progressively lower until it reaches a target situated 2.54 cm beneath the starting plane. The temperature of the bath, read at that moment on a thermometer having its bulb at the height of the test-sample, represents the softening point of the bitumen.

Table 4 indicates the experimental range considered (i.e. the minimum value and maximum value for each parameter in the 36 samples and the relative average) and the results obtained with the neural network in the training phase of the neural network itself (see last column of table 4).

The trained neural network was subjected to a subsequent validation of the correlation algorithm. For this purpose, the network was used in predictive mode by feeding, as input data, the data relating to 15 unknown samples of bitumen.

The same physico-chemical parameters used for the training were adopted as input data, excluding the values of the softening point. The results relating to the softening point, returned by the neural network, were compared with the corresponding values determined experimentally. As can be observed in Table 5, on an average the absolute error, expressed as difference between the value measured experimentally and that calculated by the neural network, in the estimation of the inversion point, is 0.9, an extremely good value considering that the repeatability of the analytical method used is 1.

On the basis of this prediction for the specific process, it is possible to effect the ranking of the crude-oils and blends.

**Table 2 - Experimental range considered and results with the neural network on the training set (69 samples)**

| | INPUT | | | | | OUTPUT | Neural network |
|---|---|---|---|---|---|---|---|
| | API | Sulfur (%)* | Viscosity @50°C (VBN) | Asphaltenes (%)* | Penetration @25°C (dmm) | Quantity PPA phase inversion (%)** | Absolute Error* |
| Min val | 3.70 | 2.57 | 39.59 | 4.30 | 25 | 13 | 0.01 |
| Max val | 13.70 | 5.06 | 48.82 | 12.88 | 298 | 34 | 2.39 |
| Average | 10.24 | 3.47 | 42.16 | 7.08 | 175 | 16 | 0.68 |
| R² | | | | | | | 0.9814 |
| Percentage reply with error < 1 | | | | | | | 72.5 |
| Percentage reply with error < 2 | | | | | | | 94.2 |

| | | | | | | | |
|---|---|---|---|---|---|---|---|
| *: values expressed as weight percentage referring to the total weight of the residue from which the bitumen derives. **: weight percentage referring to the total weight of the modified bitumen. | | | | | | | |

**Table 3 - Experimental range considered and prediction results on a set of 19 unknown samples.**

| | INPUT | | | | | OUTPUT | Neural network |
|---|---|---|---|---|---|---|---|
| | API | Sulfur (%)* | Viscosity @50°C (VBN) | Asphaltenes (%)* | Penetration @25°C (dmm) | Quantity PPA phase inversion (%)** | Absolute Error* |
| Min val | 8.42 | 2.99 | 39.85 | 5.60 | 132 | 13 | 0.01 |
| Max val | 12.23 | 4.26 | 43.61 | 8.75 | 224 | 22 | 1.48 |
| Average | 10.64 | 3.65 | 41.33 | 6.52 | 187 | 17 | 0.63 |
| R² | | | | | | | 0.9681 |
| Percentage reply with error < 1 | | | | | | | 71.4 |
| Percentage reply with error < 2 | | | | | | | 100.0 |

| | | | | | | | |
|---|---|---|---|---|---|---|---|
| *: values expressed as weight percentage referring to the total weight of the residue from which the bitumen derives. **: weight percentage referring to the total weight of the modified bitumen. | | | | | | | |

**Table 4 - Experimental range considered and results with the neural network on the training set (36 samples)**

| | INPUT | | | | | OUTPUT | Neural network |
|---|---|---|---|---|---|---|---|
| | API | Viscosity @50°C (VBN) | CCR (%)* | Asphaltenes (%)* | Penetration @25° C (dmm ) | Softening point (°C) | Absolute Error |
| Min val | 12.2 | 40.31 | 13.83 | 5.71 | 176 | 45 | 0.02 |
| Max val | 10.9 | 40.78 | 14.12 | 5.83 | 202 | 39 | 7.57 |
| Average | 9.7 | 43.08 | 16.23 | 6.56 | 180 | 44 | 1.02 |
| R² | | | | | | | 0.6740 |
| Percentage reply with error < 1 | | | | | | | 61.9 |
| Percentage reply with error < 2 | | | | | | | 95.2 |

| | | | | | | | |
|---|---|---|---|---|---|---|---|
| *: values expressed as weight percentage referring to the total weight of the residue from which the bitumen derives. | | | | | | | |

**Table 5 - Experimental range considered and prediction results on a set of 15 unknown samples.**

| | INPUT | | | | | OUTPUT | Neural network |
|---|---|---|---|---|---|---|---|
| | API | Viscosity @50°C (VBN) | CCR (%)* | Asphaltenes (%)* | Penetration @25° C (dmm ) | Softening point (°C) | Absolute Error |
| Min val | 10.80 | 3.87 | 39.85 | 6.34 | 41 | 38 | 0.15 |
| Max val | 9.40 | 4.04 | 42.58 | 6.53 | 43 | 45 | 2.73 |
| Average | 12.20 | 2.99 | 40.31 | 5.71 | 44 | 42 | 0.92 |
| R² | | | | | | | 0.8826 |
| Percentage reply with error < 1 | | | | | | | 53.3 |
| Percentage reply with error < 2 | | | | | | | 93.3 |

| | | | | | | | |
|---|---|---|---|---|---|---|---|
| *: values expressed as weight percentage referring to the total weight of the residue from which the bitumen derives. | | | | | | | |

## Claims

1. A method for predicting a parameter of a bitumen having a penetration nP at 25°C, said bitumen being obtained by means of a process (P) applied to a hydrocarbon and/or a blend of hydrocarbons, comprising the following operative phases: a) obtaining, through experimental analyses and/or databases and/or blending models, the values of N physico-chemical parameters of the hydrocarbon or blend of hydrocarbons;
a1) preparing a neural network and training it in order to obtain a trained neural network, according to the following operative phases:
i) obtaining by means of experimental analyses and/or databases and/or blending models, the values of N' physico-chemical parameters of a number I of hydrocarbons and/or a number MI of hydrocarbon blends;
ii) obtaining by means of experimental analyses the penetration nP values of each bitumen resulting from the application of the process P to the I hydrocarbons
and/or MI blends of hydrocarbons;
iii) obtaining by means of experimental analyses the value of the parameter to be predicted for each bitumen resulting from the application of the process P to I hydrocarbons and/or MI blends of hydrocarbons;
iv) constructing an input matrix relating to the I hydrocarbons and/or MI blends of hydrocarbons comprising the values obtained in phases i) to iii),
v) training the neural network with the input matrix obtained in phase iv) to obtain a correlation between the physico-chemical parameters of the I hydrocarbons and/or MI blends of hydrocarbons and the nP values of the bitumens resulting from the application of the process P to the I hydrocarbons and/or MI blends of hydrocarbons, and the parameter values to be predicted, thus obtaining a trained neural network.
b) feeding the values of the N parameters obtained in step a) together with the nP value of the bitumen, to the trained neural network;
c) predicting the parameter of the bitumen on the basis of the result of the correlation obtained by the neural network during step b).

2. The method according to claim 1, wherein the hydrocarbons are crude oils and/or distillation residues of crude oils.

3. The method according to any of the claims from 1 to 2, wherein the parameter to be predicted is a phase compatibility index of the bitumen with a polymeric modifying agent.

4. The method according to claim 3, wherein the polymeric modifying agent is PPA or SBS, preferably PPA.

5. The method according to claim 3 or 4, wherein the parameter to be predicted is the phase inversion point of the bitumen with the polymeric modifying agent.

6. The method according to any of the claims from 1 to 2, wherein the parameter to be predicted is the softening point of the bitumen.

7. The method according to any of the claims from 1 to 6, wherein **N** is an integer varying from 3 to 10, preferably from 4 to 7.

8. The method according to any of the claims from 1 to 7, wherein **N'** is an integer varying from 3 to 10, preferably from 4 to 7.

9. The method according to any of the claims from 1 to 8, wherein I and MI are integers, the same or different, varying from 10 to 90, preferably from 20 to 90.

10. The method according to any of the claims from 1 to 9, wherein the physico-chemical parameters are selected from those indicated in the characterization crude assays of crude oils.

11. The method according to any of the claims from 1 to 10, wherein the physico-chemical parameters comprise one or more of the following parameters: density, sulphur content and/or metals content and/or asphaltenes content, viscosity, CCR.

12. The method according to any of the claims from 1 to 11, wherein the neural network, during the training, is optimized by means of genetic algorithms.

## Patentansprüche

1. Verfahren für die Prognose eines Parameters von einem Bitumen mit einer Penetration nP bei 25 °C, wobei das Bitumen mittels eines an einem Kohlenwasserstoff und/oder einem Blend von Kohlenwasserstoffen angewendeten Verfahrens (P) erhalten wird, umfassend die folgenden operativen Phasen:
a) Erhalten, durch experimentelle Analysen und/oder Datenbanken und/oder Mischmodelle, der Werte von N physiko-chemischen Parametern des Kohlenwasserstoffs oder Blends von Kohlenwasserstoffen;
a1) Herstellen eines neuralen Netzwerks und Trainieren davon, um ein trainiertes neurales Netzwerk zu erhalten, gemäß den folgenden operativen Phasen:
i) Erhalten, mittels experimenteller Analysen und/oder Datenbanken und/oder Mischmodellen, die Werte von N' physiko-chemischen Parametern einer Anzahl I von Kohlenwasserstoffen und/oder einer Anzahl MI von Kohlenwasserstoff-Blends;
ii) Erhalten, mittels experimenteller Analysen, die Penetration nP-Werte von jedem Bitumen, das aus der Anwendung des Verfahrens P an die I Kohlenwasserstoffe und/oder MI Blends von Kohlenwasserstoffen resultiert;
iii) Erhalten, mittels experimenteller Analysen, den Wert des zu prognostizierenden Parameters für jedes Bitumen, das aus der Anwendung des Verfahrens P an die I Kohlenwasserstoffe und/oder MI Blends von Kohlenwasserstoffen resultiert;
iv) Errichten einer Input-Matrix, die sich auf die I Kohlenwasserstoffe und/oder MI Blends von Kohlenwasserstoffen bezieht, umfassend die in Phasen i) bis iii) erhaltenen Werte,
v) Trainieren des neuralen Netzwerks mit der in Phase iv) erhaltenen Input-Matrix, um eine Korrelation zwischen physiko-chemischen Parametern der I Kohlenwasserstoffe und/oder MI Blends von Kohlenwasserstoffen und der nP Werte der Bitumene, die aus der Anwendung des Verfahrens P an die I Kohlenwasserstoffe und/oder MI Blends von Kohlenwasserstoffen resultieren, und der zu prognostizierenden Parameterwerte zu erhalten, dadurch Erhalten eines trainierten neuralen Netzwerks,
b) Zuführen der Werte der in Schritt a) erhaltenen N Parameter zusammen mit dem nP Wert des Bitumens, zu dem trainierten neuralen Netzwerk;
c) Prognostizieren des Parameters des Bitumens basierend auf dem Ergebnis der durch das neurale Netzwerk während Schritt b) erhaltenen Korrelation.

2. Verfahren nach Anspruch 1, wobei die Kohlenwasserstoffe Rohöle und/oder Destillationsrückstände von Rohölen sind.

3. Verfahren nach einem beliebigen der Ansprüche 1 bis 2, wobei der zu prognostizierende Parameter ein Phasenkompatibilitäts-Index des Bitumens mit einem Polymer-modifizierenden Mittel ist.

4. Verfahren nach Anspruch 3, wobei das Polymer-modifizierende Mittel PPA oder SBS, vorzugsweise PPA, ist.

5. Verfahren nach Anspruch 3 oder 4, wobei der zu prognostizierende Parameter der Phasen-Inversionspunkt des Bitumens mit dem Polymer-modifizierenden Mittel ist.

6. Verfahren nach einem beliebigen der Ansprüche 1 bis 2, wobei der zu prognostizierende Parameter der Erweichungspunkt des Bitumens ist.

7. Verfahren nach einem beliebigen der Ansprüche 1 bis 6, wobei N eine Ganzzahl ist, die von 3 bis 10, vorzugsweise von 4 bis 7, variiert.

8. Verfahren nach einem beliebigen der Ansprüche 1 bis 7, wobei N' eine Ganzzahl ist, die von 3 bis 10, vorzugsweise von 4 bis 7, variiert.

9. Verfahren nach einem beliebigen der Ansprüche 1 bis 8, wobei I und MI Ganzzahlen sind, gleich oder verschieden, die von 10 bis 90, vorzugsweise von 20 bis 90, variieren.

10. Verfahren nach einem beliebigen der Ansprüche 1 bis 9, wobei die physiko-chemischen Parameter aus denen in den Charakterisierungs-Roh-Assays von Rohölen angegebenen ausgewählt sind.

11. Verfahren nach einem beliebigen der Ansprüche 1 bis 10, wobei die physicochemischen Parameter einen oder mehrere der folgenden Parameter umfassen: Dichte, Schwefelgehalt und/oder Metallgehalt und/oder Asphaltstoffgehalt, Viskosität, CCR.

12. Verfahren nach einem beliebigen der Ansprüche 1 bis 11, wobei das neurale Netzwerk, während dem Training, mittels genetischer Algorithmen optimiert wird.

## Revendications

1. Procédé visant à prédire un paramètre d'un bitume présentant un indice de pénétration nP à 25°C, lequel bitume est obtenu au moyen d'un procédé (P) appliqué à un hydrocarbure et/ou à un mélange d'hydrocarbures, lequel procédé comporte les étapes opératoires suivantes :
a) obtenir, par l'intermédiaire d'analyses expérimentales et/ou de bases de données et/ou de modèles de mélange, les valeurs de N paramètres physico-chimiques de l'hydrocarbure ou du mélange d'hydrocarbures ;
a1) préparer un réseau neuronal et l'entraîner afin d'obtenir un réseau neuronal entraîné, en effectuant les étapes opératoires suivantes :
i) obtenir, par l'intermédiaire d'analyses expérimentales et/ou de bases de données et/ou de modèles de mélange, les valeurs de N' paramètres physico-chimiques d'un nombre I d'hydrocarbures et/ou d'un nombre MI de mélanges d'hydrocarbures ;
ii) obtenir, au moyen d'analyses expérimentales, les valeurs des indices de pénétration nP de chaque bitume résultant de l'application du procédé P aux I hydrocarbures et/ou aux MI mélanges d'hydrocarbures ;
iii) obtenir, au moyen d'analyses expérimentales, la valeur du paramètre à prédire pour chaque bitume résultant de l'application du procédé P aux I hydrocarbures et/ou aux MI mélanges d'hydrocarbures ;
iv) construire une matrice d'entrée concernant les I hydrocarbures et/ou les MI mélanges d'hydrocarbures et comprenant les valeurs obtenues dans les étapes (i) à (iii) ;
v) et entraîner le réseau neuronal avec la matrice d'entrée obtenue dans l'étape (iv), pour obtenir une corrélation entre les paramètres physico-chimiques des I hydrocarbures et/ou des MI mélanges d'hydrocarbures et les valeurs de l'indice nP des bitumes résultant de l'application du procédé P aux I hydrocarbures et/ou aux MI mélanges d'hydrocarbures et les valeurs du paramètre à prédire, obtenant par là un réseau neuronal entraîné ;
b) fournir au réseau neuronal entraîné les valeurs des N paramètres obtenues dans l'étape (a), ainsi que la valeur de l'indice nP du bitume ;
c) et prédire le paramètre du bitume, en se basant sur le résultat de la corrélation obtenu par le réseau neuronal au cours de l'étape (b).

2. Procédé conforme à la revendication 1, dans lequel les hydrocarbures sont des huiles brutes et/ou des résidus de distillation d'huiles brutes.

3. Procédé conforme à l'une des revendications 1 à 2, dans lequel le paramètre à prédire est un indice de compatibilité de phase du bitume avec un agent modificateur polymère.

4. Procédé conforme à la revendication 3, dans lequel l'agent modificateur polymère est un PPA ou un SBS, de préférence un PPA.

5. Procédé conforme à la revendication 3 ou 4, dans lequel le paramètre à prédire est le point d'inversion de phases du bitume avec l'agent modificateur polymère.

6. Procédé conforme à l'une des revendications 1 à 2, dans lequel le paramètre à prédire est le point de ramollissement du bitume.

7. Procédé conforme à l'une des revendications 1 à 6, dans lequel le nombre N est un nombre entier qui vaut de 3 à 10, et de préférence, de 4 à 7.

8. Procédé conforme à l'une des revendications 1 à 7, dans lequel le nombre N' est un nombre entier qui vaut de 3 à 10, et de préférence, de 4 à 7.

9. Procédé conforme à l'une des revendications 1 à 8, dans lequel les nombres I et MI sont des nombres entiers, identiques ou différents, qui valent de 10 à 90, et de préférence, de 20 à 90.

10. Procédé conforme à l'une des revendications 1 à 9, dans lequel les paramètres physico-chimiques sont choisis parmi ceux indiqués dans les essais de caractérisation d'huiles brutes.

11. Procédé conforme à l'une des revendications 1 à 10, dans lequel les paramètres physico-chimiques comprennent l'un ou plusieurs des suivants : densité, teneur en soufre et/ou teneur en métaux et/ou teneur en asphaltènes, viscosité, indice CCR.

12. Procédé conforme à l'une des revendications 1 à 11, dans lequel le réseau neuronal, au cours de son entraînement, est optimisé au moyen d'algorithmes génétiques.
